# FASCICULE DE BREVET EUROPEEN

(11) **EP 2 181 703 B1**
(45) Date de publication et mention de la délivrance du brevet: **18.05.2016**
(21) Numéro de dépôt: 09174257.7
(22) Date de dépôt: 27.10.2009
(51) Int. Cl.: A61K 8/99, A61Q 19/00

(54) **Utilisation d'un lysat de microorganisme pour le traitement des peaux grasses**
Verwendung eines Mikroorganismen-lysats zur Behandlung von fettiger Haut
Use of a microorganism lysate for treating greasy skin

(30) Priorité: 28.10.2008 FR 0857336; 05.11.2008 US 111432 P
(43) Date de publication de la demande: 05.05.2010
(73) Titulaire: L'Oréal, 75008 Paris (FR)
(72) Inventeur: Gueniche, Audrey, 92500, Rueil Malmaison (FR); Castiel, Isabelle, 06200, Nice (FR); Bernard, Dominique, 92170, Vanves (FR)
(74) Mandataire: Le Coupanec, Pascale A.M.P.

(56) Documents cités:
- EP-A- 1 110 555
- FR-A- 2 912 917
- US-A1- 2006 008 453

## Description

La présente invention concerne le domaine des produits cosmétiques et/ou dermatologiques, plus particulièrement destinés au soin de la peau grasse.

En particulier, la présente invention vise à proposer l'utilisation d'un nouvel actif pour prévenir et/ou traiter les désordres associés à une peau grasse notamment par une action de diminution de la sécrétion du sébum.

Le sébum constitue normalement un agent hydratant de l'épiderme.

Il est le produit naturel de la glande sébacée qui constitue une annexe de l'unité pilosébacée. II s'agit essentiellement d'un mélange plus ou moins complexe de lipides. Classiquement, la glande sébacée produit du squalène, des triglycérides, des cires aliphatiques, des cires de cholestérol et, éventuellement du cholestérol libre (Stewart, M. E., Semin Dermatol 11, 100-105(1992)). L'action des lipases bactériennes convertit une part variable des triglycérides formés en acides gras libres.

Le sébocyte constitue la cellule compétente de la glande sébacée. La production de sébum est associée à un programme de différenciation terminale de cette cellule. Durant cette différenciation, l'activité métabolique du sébocyte est essentiellement axée sur la biosynthèse des lipides (la lipogenèse) et plus précisément sur la néosynthèse d'acide gras.

Une peau grasse hyper-séborrhéique est caractérisée par une sécrétion et une excrétion exagérées de sébum. Classiquement, un taux de sébum supérieur à 200 µg/cm² mesuré au niveau du front est considéré comme caractéristique d'une telle peau grasse. Une telle peau est en outre souvent associée à un défaut de desquamation, un teint luisant, un grain de peau épais, manifestations ressenties comme des imperfections cutanées ou désordres esthétiques.

Outre son aspect disgracieux, elle constitue un terrain sur lequel peuvent survenir des complications. Elle atteint les zones où les glandes sébacées sont nombreuses et résulte principalement d'une surstimulation androgénique de la production sébacée par ces glandes spécifiques.

Ainsi, l'hyperséborrhée peut également participer à la survenue des lésions d'acné vulgaire.

L'acné vulgaire est une maladie multifactorielle qui atteint la peau riche en glandes sébacées (visage, région scapulaire, bras et régions intertrigineuses). Elle est l'une des dermatoses les plus fréquentes.

Sous sa forme la plus légère, cette dermatose concerne presque chaque être humain. Sa fréquence est maximale à l'âge de la puberté, mais elle peut se manifester pour la première fois dès l'âge de 7 à 9 ans et jusqu'à des âges dépassant 40 ans. Elle touche en outre aussi bien les hommes que les femmes.

Parmi ses formes les plus fréquentes, on peut citer les acnés comédoniennes, communément appelées acnés juvéniles, les acnés papulo-pustuleuses et/ou nodulaires, l'acné conglobata et l'acné « exogène » apparaissant en réaction à des facteurs extérieurs inflammatoires.

Plus précisément, l'acné est une maladie du follicule de la glande sébacée. Les cinq facteurs pathogéniques suivants jouent un rôle déterminant dans la constitution de l'acné:
- prédisposition génétique,
- surproduction de sébum (séborrhée),
- androgène,
- troubles de la kératinisation folliculaire (comédogenèse), et
- colonisation bactérienne et facteurs inflammatoires.

En effet, dans les parties les plus profondes de la portion infundibulaire du follicule pileux, on constate la formation d'une quantité de kératinocytes supérieure à la normale. Ces cellules se différencient en cellules cornées qui obstruent progressivement la lumière du canal folliculaire. Le processus physiologique de desquamation continue de l'acro-infundibulum vers la surface est perturbé par l'adhérence augmentée des cellules cornées produites. Il se forme un bouchon hyperkératosique constituant le comédon, lésion initiale de l'acné. Enfin les trois germes locaux prédominants, le *Staphyloccus epidermidis*, le *Malassezia furfur* et le *Propionibaeterium acnes* trouvent au niveau du follicule sébacé un milieu nutritif idéal. L'altération du milieu et l'amélioration des conditions de croissance de la microflore entraînent une augmentation de produits pro inflammatoires tels que lipases, protéases, et interleukines. On admet que les lipases produites dissocient les triglycérides en acides gras libres, lesquels agissant comme des irritants pour l'épithélium folliculaire, stimulent ultérieurement l'hyperprolifération. A la faveur de l'intensification du processus inflammatoire, les granulocytes sont attirés et migrent dans la lumière du follicule où ils contribuent finalement à la rupture enzymatique de la paroi folliculaire.

Les manifestations cliniques dites de rétention constatées peuvent être de type comédon ouvert ou fermé (microkyste, microcomédon, tête blanche). Les lésions inflammatoires dérivées des lésions de rétention peuvent être de type papules, pustules, avec des nodules indurés, des abcès, des fistules, des états cicatriciels.

Ainsi, les sujets acnéiques et à tendance acnéique possèdent le plus souvent une peau grasse, à tendance grasse ou mixte. Leur peau est le plus souvent luisante avec de nombreuses imperfections entre autre du visage (microkyste, micro comédon, tête blanche, papules, pustules, avec des nodules indurés, abcès, fistules, des états cicatriciels). Les imperfections peuvent être aussi de type peau terne, brouillée, dyschromies, rougeurs, peaux rugueuses avec des plaques de peaux sèches. Il est constaté une hyperkératose cutanée, au niveau du visage les pores sont dilatés, la peau souvent rugueuse avec un *stratum corneum* épais laissant percevoir en plaque des zones de peau sèche (atrophie épidermique et desquamation légère).

En conséquence, l'hyperséborrhée est manifestement un phénomène biologique qu'il apparaît important de contrôler efficacement pour prévenir la manifestation des troubles cutanés associés.

Pour lutter contre l'hyperséborrhée, il a déjà été proposé divers composés qui, par application topique sur la peau, sont susceptibles de diminuer la lipogenèse au niveau des sébocytes et limiter, par voie de conséquence, la production de sébum.

Malheureusement, les traitements actuellement disponibles ne sont pas totalement satisfaisants, notamment au regard des effets secondaires qui leur sont fréquemment associés tels qu'irritatifs avec certains topiques comme les rétinoïdes et benzoylperoxydes, voire gastro-intestinaux (antibiothérapie orale)). En outre, il est fréquemment observé des résistances de *P. acnes* à certaines thérapies antibactériennes locales.

Il demeure donc un besoin de disposer de nouveaux actifs susceptibles d'exercer une action cosmétique ou thérapeutique bénéfique sur les peaux grasses ou à tendance grasse.

Les peaux grasses considérées dans l'invention peuvent être des peaux grasses acnéiques ou des peaux grasses non acnéiques.

Egalement une peau grasse considérée dans la présente invention peut être une peau grasse associée ou non associée à une peau infectée.

Il subsiste également un besoin de disposer d'actifs permettant de rétablir l'écoflore des peaux grasses.

Il existe également un besoin de disposer de nouvelles compositions efficaces pour prévenir et/ou traiter les peaux grasses ou à tendance grasse et qui soient agréables et confortables à mettre en oeuvre, favorisant ainsi l'observance du traitement.

Il existe également un besoin de disposer de nouveaux actifs permettant de prévenir et/ou traiter les désordres des peaux grasses notamment tels que les dermites séborrhéiques et en particulier l'acné.La présente invention a pour objet de satisfaire ces besoins.

Ainsi, selon un premier objet, l'invention concerne l'utilisation cosmétique d'une quantité efficace d'un lysat d'au moins un microorganisme du genre *Bifidobacterium longum.*

Selon un mode de réalisation, l'invention concerne les peaux grasses acnéiques ou non acnéiques, et de préférence non acnéiques.

Selon un autre mode de réalisation, l'invention concerne les peaux grasses non infectées, acnéiques ou non acnéiques, et de préférence non acnéiques.

De telles peaux se caractérisent, notamment, par des imperfections se traduisant par une peau terne ou brouillée, et présentant éventuellement des dyschromies, des rougeurs, un aspect rugueux, ou des plaques de peaux sèches.

Les inventeurs ont en effet constaté qu'un tel lysat s'avère efficace pour la prévention et/ou le traitement des désordres associés à la peau grasse et/ou à tendance grasse.

Au sens de la présente invention, on entend par « peau », la peau du visage ou du corps.

Par peau grasse « non-acnéique », on entend au sens de la présente invention désigner une peau présentant une sécrétion et une excrétion excessive de sébum, par exemple liées à un déséquilibre hormonal ou un trouble endocrinien, ou à des facteurs environnementaux tels que les milieux chauds et humides, ou à la prise de tabac, au stress, à la pollution ou à l'eau calcaire, et dénuée des signes d'acné notamment tels que définis ci-après.

Par « quantité efficace », on entend, au sens de la présente invention, une quantité suffisante pour obtenir l'effet attendu.

Au sens de la présente invention, le terme « prévenir » entend le fait de diminuer le risque de survenue de la manifestation du désordre considéré.

A la connaissance des inventeurs, cette efficacité d'un lysat de microorganismes du genre Bifidobacterium longum n'a jamais été décrite.

On connaît certes de EP 0 043 128, la mise en oeuvre d'un lysat de microorganisme tel que le lysat Repair Complex CLR, mais uniquement à des fins de réparation de l'ADN des cellules de la peau.

Pour leur part, les documents suivants proposent la mise en oeuvre de microorganismes et pour l'essentiel à des fins de traitement de peaux sèches et/ou sensibles et troubles associés mais sous une forme distincte d'un lysat.

Ainsi, le document WO 02/28402 décrit que des microorganismes probiotiques peuvent avoir un effet bénéfique dans la régulation de réactions d'hypersensibilité cutanée comme les réactions inflammatoires et allergiques qui relèvent d'un processus immunologique. Il est également rapporté dans « Probiotics in the managment of atopic eczema, Clinical and Expérimental Allergy 2000 », Volume 30, pages 1604-1610, une étude concernant l'effet de probiotiques sur les mécanismes immunitaires infantiles comme par exemple la dermatite atopique. Le document US 5,756,088, décrit, quant à lui, un régime alimentaire, comprenant notamment un acide gras polyinsaturé et/ou de la biotine, et un Bifidobacterium, ayant des effets prophylactiques et thérapeutiques sur les dermatoses animales. Les documents EP 1 609 463, EP 1 642 570, EP 1 731 137 et FR 2 876 029 décrivent des compositions associant un ou plusieurs microorganisme(s) probiotique(s) à un cation minéral pour le traitement des peaux sensibles. Quant au document PCT/FR2006/050768, il propose pour le traitement des peaux sensibles associées à une peau sèche, une association d'un microorganisme probiotique avec un acide gras polyinsaturé et/ou ester d'acide gras polyinsaturé.

En conséquence, aucun de ces documents ne décrit la mise en oeuvre d'un microorganisme du genre Bifidobacterium longum et encore moins d'un lysat conforme à l'invention, en tant qu'actif utile pour le traitement et/ou la prévention de la peau grasse et/ou à tendance grasse et des désordres cutanés associés.

L'invention a également pour objet l'utilisation cosmétique, de préférence par voie topique, d'une quantité efficace d'un lysat d'au moins un microorganisme du genre Bifidobacterium longum à titre d'actif, destiné à prévenir et/ou traiter les dermatoses séborrhéiques associées aux peaux grasses ou à tendance grasse.

Il peut notamment s'agir d'une acné.

Selon un autre mode de réalisation, l'invention vise à prévenir et/ou traiter les dermatoses séborrhéiques associées aux peaux grasses ou à tendance grasse non acnéiques.

La présente invention vise également l'utilisation cosmétique du lysat précité à titre d'actif pour prévenir et/ou traiter les lésions et/ou imperfections de la peau grasse ou à tendance grasse et en particulier les lésions de rétention de type comédon ouvert ou fermé (microkyste, microcomédon, têtes blanches) et/ou les imperfections de type peau terne, luisante ou brouillée, dyschromie, rougeurs, peaux rugueuses, avec, le cas échéant, des plaques de peaux sèches.

Selon un mode de réalisation particulier, l'invention a pour objet l'utilisation du lysat précité pour la préparation d'une composition notamment dermatologique destinée à prévenir et/ou traiter la peau grasse ou à tendance grasse et les désordres associés, comme par exemple les dermatoses, notamment de type séborrhéiques, et en particulier l'acné.

Elle vise en particulier l'utilisation d'un tel lysat pour la préparation d'une composition notamment dermatologique, destinée au traitement ou à la prévention de l'acné, et en particulier de l'acné comédonienne, papulo-pustuleuse et/ou nodulaire, l'acné conglobata et l'acné exogène.

De telles dermatoses peuvent être le fait d'affection bénigne provoquées par la prolifération excessive d'un champignon et/ou de levure et notamment de levure du genre *Malassezia.*

Selon un autre mode de réalisation particulier, l'invention a pour objet l'utilisation du lysat précité pour la préparation d'une composition notamment dermatologique destinée à prévenir et/ou traiter la peau grasse ou à tendance grasse non-acnéique et les désordres associés, comme par exemple les dermatoses, notamment de type séborrhéiques, distinctes de l'acné.

Or, comme il ressort des données présentées dans les exemples, les inventeurs ont notamment caractérisé l'aptitude de certains de ces microorganismes à stimuler la synthèse d'un nombre surprenant de protéines susceptibles de favoriser et renforcer les défenses antimicrobiennes de l'épiderme.

En particulier, les inventeurs ont démontré qu'un lysat de *Bifidobacterium longum* permettait de stimuler l'expression de différentes protéines de défenses antimicrobiennes de l'épiderme telles que la Ribonucléase 7 (n° accession Uniref Q9H1E1), la dermcidine (P81605), la «prolactin-inducible protein» (P12273), les protéines S100 A8 et A9 (P05109 et P06702), et la protéine histone (Q5R2W0), aptes à renforcer les défenses de l'épiderme contre la colonisation excessive de microorganismes pathogènes.

Or, cette stimulation des protéines précitées a pour avantage de s'opposer efficacement à une colonisation de l'épiderme par les microorganismes *Malassezia furfur* et *Propionibacterium acnes* responsables des désordres cutanés associés à la peau grasse et/ou à tendance grasse. Cette diminution obtenue à l'aide du lysat selon l'invention contribue donc à rétablir une écoflore équilibrée avec pour conséquence une diminution des états inflammatoires de la peau et une régulation de la séborrhée. Par voie de conséquence, les imperfections se réduisent, le teint s'éclaire devient plus homogène, sans zones de dyschromies, ni de sécheresse.

Un traitement, conforme à l'invention, peut se révéler d'autant plus efficace sur l'acné et les imperfections du visage que le lysat associe à ses propriétés de stimulation des éléments de défense épidermique des propriétés de stimulation de la synthèse de protéases impliquées dans le phénomène de desquamation (KLK7 (P49862), KLK5 (Q9Y337), Cathepsin L2 (060911)) comme en témoigne l'augmentation de fragments de protéines du cornéodesmosomes (DSG1 (Q02413), DSCla (Q9HB01), Cdsn (Q15517) induite par le lysat. Le bouchon kératinique du comédon peut, semble-t-il, être alors éliminé rapidement par l'action de ces enzymes protéolytiques, évitant la création d'un milieu fermé propice au développement bactérien et à une inflammation consécutive.

La présente invention vise également l'utilisation d'un lysat conforme à l'invention pour la préparation d'une composition notamment dermatologique destinée à réguler la séborrhée.

Elle vise en outre l'utilisation cosmétique d'une quantité efficace d'un lysat d'au moins un microorganisme du genre Bifidobacterium longum et/ou une de ses fractions, à titre d'actif, destiné à maintenir et/ou restaurer l'homéostasie de la peau.

Une utilisation conforme à l'invention peut, en outre, comprendre la mise en oeuvre d'un lysat d'au moins un microorganisme du genre Bifidobacterium longum en association avec une quantité efficace d'au moins un microorganisme notamment probiotique annexe, distinct dudit lysat.

Au sens de l'invention, l'expression « distinct dudit lysat » signifie qu'il est possible de distinguer au sein de la composition soit deux microorganismes différents soit deux formes différentes d'un même microorganisme. Ainsi, lorsque le microorganisme annexe est du genre Bifidobacterium longum et correspond à la même espèce que celle figurant le lysat requis selon l'invention, ce microorganisme annexe est alors présent sous une forme autre qu'un lysat, par exemple sous une forme vivante.

Une utilisation conforme à la présente invention peut, en outre, comprendre la mise en oeuvre d'un lysat d'au moins un microorganisme du genre Bifidobacterium longum et en association avec une quantité efficace d'au moins un actif destiné à diminuer et/ou corriger la sécrétion excessive de sébum, par exemple un actif anti-séborrhéique notamment tel que décrit ci-après.

Selon un autre de ses aspects, la présente invention concerne une composition cosmétique et/ou dermatologique, utile pour prévenir et/ou traiter la peau grasse ou à tendance grasse et les désordres esthétiques associés, comprenant dans un milieu physiologiquement acceptable, au moins une quantité efficace d'un lysat d'au moins un microorganisme du genre Bifidobacterium longum, en association avec au moins un actif anti-séborrhéique, notamment tel que décrit ci-après.

Selon un autre de ses aspects, l'invention a pour objet un procédé notamment cosmétique pour prévenir et/ou traiter la peau grasse ou à tendance grasse et les désordres cutanés associés notamment esthétiques chez un sujet comprenant au moins une étape d'administration audit sujet d'une quantité efficace d'un lysat d'au moins un microorganisme du genre Bifidobacterium longum.

Selon une variante de réalisation de l'invention, un lysat selon l'invention peut être mis en oeuvre par voie orale.

Selon une autre variante de réalisation de l'invention, le lysat selon l'invention peut être mis en oeuvre par voie topique.

Comme précisé ci-après, les compositions le contenant sont formulées pour être compatibles avec le mode d'administration retenu.

### Lysat de microorganisme(s)

Comme précisé précédemment, les microorganismes du genre Bifidobacterium longum utilisés à titre d'actifs selon l'invention sont mis en oeuvre sous la forme d'un lysat.

Un lysat désigne communément un matériau obtenu à l'issue de la destruction ou dissolution de cellules biologiques par un phénomène dit lyse cellulaire provoquant ainsi la libération des constituants biologiques intracellulaires naturellement contenus dans les cellules du microorganisme considéré.

Au sens de la présente invention, le terme lysat est utilisé indifféremment pour désigner l'intégralité du lysat obtenu par lyse du microorganisme concerné ou seulement une fraction de celui-ci.

Ainsi, l'invention concerne la mise en oeuvre d'un lysat de Bifidobacterium longum.

Le lysat mis en oeuvre est donc formé en tout ou partie des constituants biologiques intracellulaires et des constituants des parois et membranes cellulaires.

Plus précisément, il contient la fraction cytoplasmique cellulaire renfermant les enzymes tels que la déhydrogénase d'acide lactique, les phosphatases, les phosphokétolases, transaldolases et les métabolites. A titre illustratif, les constituants des parois cellulaires sont notamment le peptidoglycane, la muréine ou mucopeptide et l'acide teichoïque et les constituants des membranes cellulaires sont composés de glycérophospho lipides.

Cette lyse cellulaire est accomplie par ultrasons.

Plus particulièrement, ce lysat peut être obtenu selon la technologie décrite dans le brevet US 4,464,362 et notamment selon le protocole suivant.

Le microorganisme considéré de type *Bifidobacterium species* est cultivé anaérobiquement dans un milieu de culture adéquat, par exemple selon les conditions décrites dans les documents US 4,464,362 et EP 0 043 128. Lorsque la phase stationnaire du développement est atteinte, le milieu de culture peut être inactivé par pasteurisation, par exemple à une température de 60 à 65 °C pendant 30 mn. Les microorganismes sont alors recueillis par une technique de séparation conventionnelle par exemple filtration membranaire, centrifugation et remis en suspension dans une solution NaCl physiologique stérile. Le lysat peut être obtenu par désintégration aux ultra-sons d'un tel milieu afin d'en libérer les fractions cytoplasmiques, les fragments de paroi cellulaire et les produits issus du métabolisme. Puis tous les composants dans leur distribution naturelle sont ensuite stabilisés dans une solution aqueuse faiblement acide.

On obtient ainsi généralement un lysat possédant une concentration de l'ordre de 0,1 à 50 %, en particulier de 1 à 20 % et notamment environ 5 % en poids en matière(s) active(s) par rapport à son poids total.

Le lysat peut être mis en oeuvre sous différentes formes, sous la forme d'une solution ou sous une forme pulvérulente.

Il peut avantageusement s'agir du lysat enregistré sous le nom INCI : Bifidat ferment Lysate, sous le nom EINECS: *Bifidobacterium longum,* sous le N° EINECS: N° 306-168-4 et sous le N° CAS : N° 96507-89-0.

Le produit commercialisé sous la dénomination Repair Complex CLR^{®} par la société K. RICHTER GmbH et qui est formé d'un lysat inactivé de l'espèce *Bifidobacterium longum,* entre dans le cadre de l'invention.

L'actif formant le lysat et appartenant au genre Bifidobacterium longum peut être formulé dans une composition à raison d'au moins 0,0001 % (exprimé en poids sec), en particulier à raison de 0,001 à 20 % et plus particulièrement à raison de 0,01 à 2 % en poids sec de matière active par rapport au poids total du support ou de la composition le contenant.

La teneur en lysat pour la voie topique selon l'invention peut varier de 0,0001 % à 30 % en poids, notamment de 0,01 à 15 % en poids, et en particulier de 0,1 à 10 % en poids par rapport au poids total de la composition le contenant.

Dans le cas particulier où le lysat est dédié à une administration par voie orale, la concentration en lysat peut être ajustée de manière à correspondre à des doses (exprimées en équivalent de microorganisme) variant de 5.10² à 10¹³ ufc/j et en particulier de 10⁵ à 10¹¹ ufc/j.

Selon une variante de l'invention, un lysat convenant à l'invention est mis en oeuvre en association avec au moins un autre microorganisme.

Ainsi, l'invention concerne l'utilisation, outre du lysat d'un microorganisme du genre *Bifidobacterium* longum d'au moins une quantité efficace d'au moins un microorganisme annexe, notamment de type probiotique, et/ou une de ses fractions, distinct dudit lysat.

Au sens de la présente invention, on entend par « microorganisme probiotique », un microorganisme vivant qui, lorsqu'il est consommé en quantité adéquate, a un effet positif sur la santé de son hôte « joint FAO/WHO Expert Consultation on Evaluation of Health and Nutritional Properties of Probiotic in Food Including Powder Milk with Live Lactic Acid Bacteria, 6 octobre 2001 », et qui peut en particulier améliorer l'équilibre microbien intestinal.

Ces microorganismes convenant à l'invention peuvent être choisis notamment parmi les ascomycetes telles que *Saccharomyces, Yarrowia, Kluyveromyces, Torulaspora, Schizosaccharomyces pombe, Debaromyces, Candida, Pichia, Aspergillus* et *Pénicillium,* des bactéries du genre *Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus et Lactobacillus* et leurs mélanges.

Comme ascomycètes convenant tout particulièrement à la présente invention, on peut en particulier citer *Yarrowia lipolitica et Kluyveromyces lactis,* de même que *Saccharomyces cereviseae, Torulaspora, Schizosaccharamyces pombe, Candida* et *Pichia.*

Des exemples spécifiques de microorganismes probiotiques sont *Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus paracasei, Lactobacillus rhamnosus (Lactobacillus GG), Lactobacillus sake, Lactococcus lactis, Streptococcus thermophilus, Staphylococccus carnosus,* et *Staphylococcus xylosus et leurs mélanges.*

Plus particulièrement, il s'agit de microorganismes probiotiques issus du groupe des bactéries lactiques, comme notamment les *Lactobacillus.*

A titre illustratif de ces bactéries lactiques, on peut plus particulièrement citer les *Lactobacillus johnsonii, Lactobacillus paracasei, Lactobacillus reuteri, Lactobacillus rhamnosus* et leurs mélanges.

Les espèces convenant tout particulièrement sont les *Lactobacillus johnsonii,* dont en particulier la souche déposée suivant le traité de Budapest avec l'Institut Pasteur (28 rue du Docteur Roux, F-75024 Paris cedex 15) sous la désignation suivante CNCM I-1225.

D'une manière générale, les compositions à application topique selon l'invention comprennent généralement de 0,0001 à 30 %, en particulier de 0,001 à 15 % et plus particulièrement de 0,1 à 10 % en un ou plusieurs microorganismes notamment probiotiques, annexes.

Ce ou ces microorganisme(s) peu(ven)t donc être inclus dans les compositions selon l'invention sous une forme vivante, semi-active ou inactivée, morte.

Dans le cas où ces microorganismes sont formulés dans une composition sous une forme vivante, la quantité de microorganismes vivants peut varier de 10³ à 10¹⁵ ufc/g, en particulier de 10⁵ à 10¹⁵ ufc/g et plus particulièrement de 10⁷ à 10¹² ufc/g de microorganismes par gramme de composition.

Dans le cas particulier où le(s) microorganisme(s) est (sont) formulé(s) dans des compositions à administrer par voie orale, la concentration en microorganisme(s) notamment probiotique(s) peut être ajustée de manière à correspondre à des doses (exprimées en équivalent de microorganisme) variant de 5.10⁵ à 10¹³ ufc/j et en particulier de 10⁷ à 10¹¹ ufc/j.

Il(s) peu(ven)t également être inclus sous forme de fractions de composants cellulaires. Le ou le(s) microorganisme(s), ou fraction(s) peu(ven)t également être introduit(s) sous la forme d'une poudre, d'un liquide, d'un surnageant de culture ou l'une de ses fractions, dilué(e) ou non, ou encore concentré(e) ou non.

Selon une variante, les compositions peuvent également contenir un cation minéral divalent.

### ACTIF

Quel que soit le mode d'administration considéré, le lysat de l'invention peut avantageusement être associé à au moins un autre actif.

Ainsi, une composition, topique ou orale, selon l'invention peut en outre contenir au moins un actif anti-séborrhéique.

Une telle formulation permet avantageusement d'amplifier les effets bénéfiques d'un lysat de l'invention.

On entend par actif anti-séborrhéique un composé capable de réguler l'activité des glandes sébacées.

Un actif anti-séborrhéique convenant à l'invention peut, notamment, être choisi parmi l'acide rétinoïque, le peroxyde de benzoyle, le soufre, la vitamine B6 (ou pyridoxine), le chlorure de sélénium, la criste marine ; les mélanges d'extrait de cannelle, de thé et d'octanoylglycine tel que le Sepicontrol A5 TEA^{®} de chez Seppic ; le mélange de cannelle, de sarcosine et d'octanoylglycine, commercialisé notamment par la société SEPPIC sous la dénomination commerciale Sepicontrol A5^{®} ; les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ; les dérivés de cuivre et en particulier le pidolate de cuivre tel que Cuivridone^{®} de Solabia ; des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ; les extraits de reine des prés (spiraea ulamaria) tel que celui vendu sous la dénomination Sébononnine^{®} par la société Silab ; les extraits d'algue Laminaria saccharina tel que celui vendu sous la dénomination Phlorogine^{®} par la société Biotechmarine ; les mélanges d'extraits de racines de pimprenelle (Sanguisorba officinalis/Poterium officinale), de rhizomes de gingembre (Zingiber officinalis) et d'écorce de cannelier (Cinnamomum cassia) tel que celui vendu sous la dénomination Sebustop^{®} par la société Solabia ; les extraits de graines de lin tel que celui vendu sous la dénomination Linumine^{®} par la société Lucas Meyer ; les extraits de Phellodendron tels que ceux vendu sous la dénomination Phellodendron extract BG par la société Maruzen ou Oubaku liquid B par la société Ichimaru Pharcos ; les mélanges d'huile d'argan, d'extrait de Serenoa serrulata (saw palmetto) et d'extrait de graines de sésame tel que celui vendu sous la dénomination Regu SEB^{®} par la société Pentapharm ; les mélanges d'extraits d'epilobe, de Terminalia chebula, de capucine et de zinc biodisponible (microalgues) tel que celui vendu sous la dénomination Seborilys^{®} par la société Green Tech ; les extraits de Pygeum afrianum tel que celui vendu sous la dénomination Pygeum afrianum sterolic lipid extract par la société Euromed ; les extraits de Serenoa serrulata tels que ceux vendus sous les dénomination Viapure Sabal par la société Actives International, ou ceux vendus par la société Euromed ; les mélanges d'extraits de plantain, de Berberis aquifolium et de salicylate de sodium tels que celui vendu sous la dénomination Seboclear^{®} par la société Rahn ; l'extrait de clou de girofle tel que celui vendu sous la dénomination Clove extract Powder par la société Maruzen ; l'huile d'argan telle que celle vendue sous la dénomination Lipofructyl^{®} par les Laboratoires Sérobiologiques ; les filtrats de protéine lactique tels que celui vendu sous la dénomination Normaseb^{®} par la société Sederma ; les extraits d'algue Laminaria, tel que celui vendu sous la dénomination Laminarghane^{®} par la société Biotechmarine ; les oligosaccharides d'algue Laminaria digitata tel que celui vendu sous la dénomination Phycosaccharide AC par la société Codif ; les extraits de sucre de canne tel que celui commercialisé sous la dénomination Policosanol^{®} par la société Sabinsa ; l'huile de schiste sulfonée, telle que celle vendue sous la dénomination Ichtyol Pale^{®} par la société Ichthyol ; les extraits d'ulmaire (spiraea ulmaria) tels que celui vendu sous la dénomination Cytobiol^{®} Ulmaire par la société Libiol ; l'acide sébacique, notamment vendu sous la forme d'un gel de polyacrylate de sodium sous la dénomination Sebosoft^{®} par la société Sederma ; les glucomannanes extraits de tubercule de konjac et modifié par des chaînes alkylsulfonates tel que celui vendu sous la dénomination Biopol Beta par la société Arch Chemical ; les extraits de Sophora angustifolia, tels que ceux vendus sous la dénomination Sophora powder ou Sophora extract par la société Bioland ; les extraits de Cinchona succirubra bark tel que celui vendu sous la dénomination le Red bark HS par la société Alban Muller ; les extraits de Quillaja saponaria tel que celui vendu sous la dénomination Panama wood HS par la société Alban Muller ; la glycine greffée sur chaîne undécylénique, telle que celle vendue sous la dénomination Lipacide UG OR par la société Seppic ; le mélange d'acide oléanolique et d'acide nordihydroguaïarétique, tel que celui vendus sous la forme d'un gel sous la dénomination AC.Net par la société Sederma ; l'acide phthalimidoperoxyhexanoïque ; le citrate de trialkyle(C₁₂-C₁₃) vendu sous la dénomination COSMACOL^{®} ECI par la société Sasol ; le citrate de trialkyle(C₁₄-C₁₅) vendu sous la dénomination COSMACOL^{®} ECL par la société Sasol ; l'acide 10-hydroxydécanoïque, et notamment les mélanges d'acide 10-hydroxydécanoïque, d'acide sébacique et de 1,10-décandiol tels que celui vendu sous la dénomination Acnacidol^{®} BG par la société Vincience ; et leurs mélanges.

L'actif anti-séborrhéique est par exemple présent dans une teneur allant de 0,1 à 10 % en poids, de préférence de 0,1 à 5 % en poids, et préférentiellement de 0,5 % à 3 % en poids, par rapport au poids total de la composition.

Outre cet actif séborrhéique, les compositions selon l'invention peuvent, en outre, contenir plusieurs autres actifs communément utilisés et/ou autorisés.

A titre d'actifs, conventionnellement mis en oeuvre, on peut citer les vitamines B3, B5, B6, B8, C, D, E, ou PP, la niacine, les caroténoïdes, les polyphénols, les minéraux et oligo-éléments, les phytooestrogènes, les protéines et les acides aminés, les mono et polysaccharides, les amino-sucres, les phytostérols et alcools triterpéniques d'origine végétale.

Les minéraux et oligo-éléments particulièrement mis en oeuvre sont le zinc, le calcium, le magnésium, le cuivre, le fer, l'iode, le manganèse, le sélénium, le chrome (III).

Parmi les polyphénols, on retient aussi en particulier les polyphénols de raisin, de thé, d'olive, de cacao, de café, de pomme, de myrtille, de sureau, de fraise, de canneberge, et d'oignon. De préférence, parmi les phytoestrogènes, on retient les isoflavones sous la forme libre ou glycosylée, telles que la génistéine, la daidzéine, la glycitéine ou encore les lignanes, en particulier ceux du lin et du schizandra chinensis.

Les acides aminés ou les peptides et les protéines les contenant, tels que la taurine, la thréonine, la cystéine, le tryptophane, la méthionine. Les lipides appartiennent de préférence au groupe des huiles contenant des acides gras mono et polyinsaturés tels que les acides oléique, linoléique, alpha-linolénique, gamma-linolénique, stéaridonique, les acides gras oméga-3 de poisson à longue chaîne, tels que l'EPA et le DHA, les acides gras conjugués issus de végétaux ou d'animaux, tels que les CLA (Conjugated Linoleic Acid).

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes, telles que les catéchines, l'hespéridine, des proanthocyanidines et des anthocyanines, l'acide lipoïque et le coenzyme Q10.

L'actif annexe peut également être au moins un prébiotique ou un mélange de prébiotiques. Plus particulièrement, ces prébiotiques peuvent être choisis parmi les oligosaccharides, produits à partir du glucose, galactose, xylose, maltose, sucrose, lactose, amidon, xylane, l'hémicellulose, l'inuline, des gommes de type acacia par exemple, ou un de leurs mélanges.

Plus particulièrement, l'oligosaccharide comprend au moins un fructo-oligosaccharide. Plus particulièrement, ce prébiotique peut comprendre un mélange de fructo-oligosaccharide et d'inuline.

Dans les formes galéniques topiques, on peut utiliser plus particulièrement comme actifs hydrophiles les protéines ou les hydrolysats de protéine, les acides aminés, les polyols, notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Pour ce qui est des actifs lipophiles, on peut utiliser le rétinol (vitamine A) et ses dérivés, le tocophérol (vitamine E) et ses dérivés, les céramides, les huiles essentielles et les insaponifiables (tocotriénol, sésamine, gamma oryzanol, phytostérols, squalènes, cires, terpènes).

On peut également associer de façon avantageuse au produit des actifs capables d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée et certains de ses dérivés; l'acide gentisique ; les oligofucoses ; l'acide cinnamique ; l'acide dioïque ; l'extrait de Saphora japonica ; le resvératrol ; les détergents et certains dérivés d'acide jasmonique ;
- et/ou sur les activités des enzymes impliquées dans la dégradation des cornéodesmosomes, telles que la stratum corneum chymotryptic enzyme (SCCE), voire d'autres protéases (trypsin-like, chymotrypsin-like, cathepsin D) ainsi que d'autres catégories d'hydrolases (ex : glycosidases, céramidases). On peut citer les agents chélatants des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP 0 852 949, ainsi que le méthyle glycine diacétate de sodium commercialisé par BASF sous la dénomination commerciale TRILON M) ; le miel ; les dérivés de sucre tels que l'O-octanoyl-6-D-maltose et la N-acétyl glucosamine ; l'urée ou certains de ses dérivés par exemple Hydrovance ; les dérivés de C-Glycosides.

### Formes galéniques

Les compositions selon l'invention peuvent se présenter sous toutes les formes galéniques normalement disponibles pour le mode d'administration retenu.

Le support peut être de nature diverse selon le type de composition considérée. Les compositions destinées à une administration par voie topique, peuvent être des solutions aqueuses, hydroalcooliques ou huileuses, de dispersions du type des solutions ou dispersions du type lotion ou sérum, d'émulsions de consistance liquide ou semi-liquide du type lait, obtenue par dispersion d'une phase grasse dans une phase aqueuse (H/E) ou inversement (E/H) ou d'une suspension ou émulsion de consistance molle, semi-solide ou solide, du type crème, de gel aqueux ou anhydre, de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de peeling, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, ou des compositions contre les piqûres d'insectes.

Les compositions selon l'invention peuvent également consister en des préparations solides constituant des savons ou des pains de nettoyage.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique. L'émulsionnant et le coémulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré et, par exemple, de 0,01 à 20 % du poids total de la composition. Ces adjuvants, selon leur nature peuvent être introduits dans la phase grasse et/ou dans la phase aqueuse.

Comme matières grasses utilisables dans l'invention, on peut citer les huiles minérales, comme par exemple, le polyisobutène hydrogéné et l'huile de vaseline, les huiles végétales, comme par exemple, une fraction liquide du beurre de karité, huile de tournesol et d'amandes d'abricot, les huiles animales, comme par exemple, le perhydrosqualène, les huiles de synthèse, notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras, comme par exemple, l'acide stéarique et, comme par exemple, des cires, notamment de paraffine, carnauba et la cire d'abeilles. On peut aussi utiliser, des composés siliconés comme les huiles siliconées et par exemple le cyclométhicone et diméthicone, les cires, les résines et les gommes siliconées.

Comme émulsionnants utilisables dans l'invention, on peut citer, par exemple, le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO^{®} par la société HENKEL, le mélange de PEG-6/PEG-32/Glycol Stéarate vendu sous la dénomination de Tefose^{®} 63 par société GATTEFOSSE, le PPG-3 myristyl éther, les émulsionnants siliconés, tels que le cétyldiméthicone copolyol et le mono- ou tristéarate de sorbitane, le stéarate de PEG-40, le monostéarate de sorbitane oxyéthyléné (20 OE).

Comme solvants utilisables dans l'invention, on peut citer les alcools inférieurs, notamment l'éthanol et l'isopropanol, le propylène glycol.

Une composition selon l'invention peut également contenir de façon avantageuse une eau thermale et/ou minérale, notamment choisie parmi l'eau de Vittel, les eaux du bassin de Vichy et l'eau de la Roche Posay.

Comme gélifiants hydrophiles, on peut citer les polymères carboxyliques tels que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305^{®} par la société SEPPIC, les polysaccharides comme les dérivés cellulosiques tels que les hydroxyalkylcelluloses, et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Dans le cas d'une utilisation par voie orale, le support est ingérable.

Le support ingérable peut être de diverses natures selon le type de composition considéré.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suppléments oraux sous forme sèche et les suppléments oraux sous forme liquide.

Le lysat de microorganisme de l'invention peut être par ailleurs formulé avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires, sont connus dans ce domaine et ne font pas ici l'objet d'une description détaillée. Pour l'ingestion, de nombreuses formes de réalisation de compositions orales et notamment de compléments alimentaires sont possibles. Leur formulation est réalisée par les procédés usuels pour produire des dragées, gélules, gels, hydrogels à libération contrôlée, émulsions, comprimés, capsules.

En particulier, le lysat de microorganisme selon l'invention peut être incorporé dans toute autre forme de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Selon un mode de réalisation particulier, les microorganismes annexes considérés selon l'invention peuvent être formulés au sein de compositions sous une forme encapsulée de manière à améliorer significativement leur durée de survie. Dans un tel cas, la présence d'une capsule peut en particulier retarder ou éviter la dégradation du microorganisme au niveau du tractus gastro-intestinal.

Le procédé de traitement cosmétique de l'invention peut être mis en oeuvre, notamment en administrant le lysat ou les compositions cosmétiques et/ou dermatologiques tels que définis ci-dessus, selon la technique d'utilisation habituelle de ces compositions. A titre illustratif, on peut procéder par applications de crèmes, de gels, de sérums, de lotions, de laits de démaquillage ou de compositions après-solaires sur la peau pour ce qui est de l'application topique.

Le procédé cosmétique selon l'invention peut être ainsi mis en oeuvre par application topique, journalière par exemple, du lysat considéré selon l'invention.

Le procédé selon l'invention peut comprendre une application unique. Selon un autre mode de réalisation, l'application est répétée par exemple 2 à 3 fois quotidiennement sur une journée ou plus et généralement sur une durée prolongée d'au moins 4, voire 1 à 15 semaines.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés avant leur mise en forme, dans l'ordre et dans des conditions facilement déterminées par l'homme de l'art.

De plus, il peut être envisagé des associations de traitement avec éventuellement des formes orales ou topiques afin de compléter ou renforcer l'activité du lysat tel que définie par l'invention.

Ainsi, on pourrait imaginer un traitement par voie topique avec une composition contenant le lysat de *Bifidobacterium* associé à une composition par voie orale ou topique contenant éventuellement un autre microorganisme probiotique ou d'autres probiotiques sous forme morte, vivante ou semi-active.

Les exemples ci-après sont présentés à titre illustratif et non limitatif du domaine de l'invention.

### EXEMPLES

**Exemple 1 : lotion pour le visage**

| Ingrédients | Quantité (%) |
|---|---|
| Lysat de *Bifidobacterium longum** | 5,00** |
| Anti-inflammatoire | 0,05 |
| Antioxydant | 0,05 |
| Isopropanol | 40,00 |
| Conservateur | 0,30 |
| Eau | Qsp 100 |

| | |
|---|---|
| * Il s'agit du lysat enregistré sous le nom INCI Bifidat ferment Lysate, et mis en oeuvre sous la forme d'une formulation à 5 % en poids d'actif. ** quantité exprimée en produit total | |

**Exemple 2 : gel pour le soin du visage**

| Ingrédients | Quantité (%) |
|---|---|
| Lysat de *Bifidobacterium longum** | 5,00** |
| Hydroxypropylcellulose (Klucel H^{®} vendu par la société HERCULES) | 1,00 |
| Vitamine E | 2,50 |
| Antioxydant | 0,05 |
| Isopropanol | 40,00 |
| Conservateur | 0,30 |
| Eau | Qsp 100 |

| | |
|---|---|
| * Il s'agit du lysat enregistré sous le nom INCI Bifidat ferment Lysate, et mis en oeuvre sous la forme d'une formulation à 5 % en poids d'actif. ** quantité exprimée en produit total | |

**Exemple 3 : lait pour le soin du visage**

| Ingrédients | Quantité (%) |
|---|---|
| Lysat de *Bifidobacterium longum** | 10,00** |
| Stéarate de glycérol | 1,00 |
| Alcool cétylstéarylique/alcool cétylstéarylique oxyéthyléné à 3 moles OE (Sinnovax AO^{®} vendu par la société HENKEL) | 3,00 |
| Alcool cétylique | 1,00 |
| Diméthicone (DC 200 Fluid^{®} vendu par la société Dow Corning) | 1,00 |
| Huile de vaseline | 6,00 |
| Myristate d'isopropyle (Estol IPM 1514^{®} vendu par la société Unichema) | 3,00 |
| Glycérine | 20,00 |
| Conservateur | 0,30 |
| Eau | Qsp 100 |

| | |
|---|---|
| * Il s'agit du lysat enregistré sous le nom INCI Bifidat ferment Lysate, et mis en oeuvre sous la forme d'une formulation à 5 % en poids d'actif. ** quantité exprimée en produit total | |

**Exemple 4 : Crème pour le soin du visage**

| Ingrédients | Quantité (%) |
|---|---|
| Arachidyl behenyl alcohol/arachidylglusoside | 3,00 |
| Isohexadecane | 7,00 |
| Lysat de *Bifidobacterium longum** | 10,00** |
| Glycérine | 2,00 |
| Extrait de *Vitreoscilla filiformis* | 3,00 |
| BHT | 0,05 |
| POB méthyle | 0,10 |
| POB propyle | 0,50 |
| Eau | Qsp 100 |

| | |
|---|---|
| * Il s'agit du lysat enregistré sous le nom INCI Bifidat ferment Lysate, et mis en oeuvre sous la forme d'une formulation à 5 % en poids d'actif. ** quantité exprimée en produit total | |

**Exemple 5 : Gel pour le soin visage**

| Ingrédients | Quantité (%) |
|---|---|
| Extrait de *Vitreoscilla filiformis* | 3,00 |
| Lysat de *bifidobacterium longum** | 10,00** |
| Antioxydant | 0,05 |
| Vitamine C | 2,50 |
| Antioxydant | 0,50 |
| Isopropanol | 40,50 |
| Conservateur | 0,30 |
| Eau | Qsp 100 |

| | |
|---|---|
| * Il s'agit du lysat enregistré sous le nom INCI Bifidat ferment Lysate, et mis en oeuvre sous la forme d'une formulation à 5 % en poids d'actif. ** quantité exprimée en produit total | |

### Exemple 6 : Résultats protéomiques

Le produit testé est un lysat de *Bifidobacterium longum* en suspension désintégrée (aux ultrasons) dans un milieu aqueux faiblement acide commercialisé sous le nom Repair Complex CLR^{®}.

L'actif a été testé seul dans une étude randomisée en double aveugle.

Soixante six femmes présentant des peaux sèches ont été réparties en deux groupes, placebo (n=33 groupe A), Repair Complex CLR^{®} (n=33 groupe B). Les traitements ont été appliqués topiquement durant 58 jours, l'actif étant formulé à 10 % de la formulation testée. Cette formule support est une émulsion H/E déminéralisée Arlacel/myrj^{®} contenant 5 % Parleam, 15 % de cyclopentasiloxane, 3 % glycérine et 2 % vaseline.

Dans la formule placebo, l'absence de lysat est compensée par de l'eau.

Les sujets ont été évalués à J1, J29, J43, et J57.

L'évolution de différents marqueurs cutanés a été étudiée par protéomique sur des échantillons de *stratum corneum* isolé.

Un prélèvement est effectué au niveau de la face externe de la jambe aux temps J1, J29, J43 et J57 par stripping vernis afin de ne prélever qu'une partie du *stratum corneum,* soit au maximum 4 à 5 couches de *stratum corneum.*

Une toile de nylon filtre 41 µm type NY41 Millipore est appliquée sur une zone préalablement définie de la jambe gauche. Puis un vernis transparent de référence 614254/T.D. comprenant : nitrocellulose 6,86 g ; isopropanol 2,94 g ; résine alkyle hypoallergénique 7,35 g ; acétyl tributyl citrate 7,7 g ; acétate d'éthyle 75,15 g ; est étalé à l'aide d'un pinceau (15 mm), puis laissée sécher pendant 15 min. La toile de nylon est ensuite récupérée à l'aide d'une pince Brucelles en arrachant d'un coup sec le stripping vernis.

Les strippings vernis sont conservés à -20 °C à plat dans des sachets plastiques.

Ces prélèvements de peau (stripping vernis de stratum corneum) ont ensuite été analysés par protéomique au moyen d'une technique dite de « marquage isobarique », pour évaluer l'expression de différentes protéines.

Cette technique dite de « marquage isobarique » ou iTRAQ repose sur le marquage des peptides triptyques avec une série de réactifs, dits isobariques car possédant tous une masse moléculaire de 145 Da, et formant un lien covalent avec les amines primaires de l'extrémité amino-terminale de la chaîne latérale des résidus lysine.

Les peptides marqués sont détectés par spectrométrie de masse avec la masse intrinsèque du peptide + 145 Da, provenant du réactif. A l'étape de fragmentation du peptide, la contribution de chacun des réactifs est appréciée par la libération d'ions (fragments) ayant des masses différentes spécifiques.

Une telle méthode est précisément décrite par Zieske (J. Exp. Bot., 2006, 57 :1501) ou Wiese et al. (Proteomics, 2007, 7 :340).

Les résultats de l'analyse par protéomique différentielle montrent que le lysat de Bifidobacterium longum stimule l'expression d'une partie des protéines de défense antimicrobienne de l'épiderme (RNase 7, Dermcidine, « Prolactin Inducible Protein », fragments d'histone, ...) et certaines protéases impliquées dans le phénomène de desquamation (KLK-7, KLK-5, cathepsine L2, fragments de protéines du cornéodesmosome).

La mise en évidence d'une stimulation des protéines précitées est un indice de la diminution de la colonisation par les microorganismes *Malassezia fulfur* et *Propionibacterium acnes* responsables des désordres cutanés associés à la peau grasse et/ou à tendance grasse.

Ainsi, une telle diminution contribue à rétablir une écoflore équilibrée qui a pour conséquence une diminution des états inflammatoires de la peau et une régulation de la séborrhée. Par voie de conséquence, les imperfections se réduisent, le teint s'éclaire devient plus homogène, sans zones de dyschromies, ni de sécheresse.

En outre, la présence de protéases, caractéristique du phénomène de desquamation, permet de traiter les zones présentant des désordres associés aux zones grasses sans provoquer de sécheresse excessive sur ces zones.

## Revendications

1. Utilisation non thérapeutique d'une quantité efficace d'un lysat obtenu par désintégration aux ultra sons d'un microorganisme de l'espèce *Bifidobacterium longum,* à titre d'actif, pour traiter et/ou prévenir les peaux grasses ou à tendance grasse et les désordres cutanés associés.

2. Utilisation selon la revendication précédente **caractérisée en ce que** les désordres cutanés peuvent être des imperfections de la peau grasse ou à tendance grasse,) et/ou des imperfections cutanées de type peau terne, luisante ou brouillée, dyschromie, rougeurs, peaux rugueuses avec, le cas échéant, des plaques de peaux sèches.

3. Utilisation d'une quantité efficace d'un lysat obtenu par désintégration aux ultra sons d'un microorganisme de l'espèce *Bifidobacterium longum,* à titre d'actif pour la préparation d'une composition destinée à réguler la séborrhée.

4. Utilisation d'une quantité efficace d'un lysat obtenu par désintégration aux ultra sons d'un microorganisme de l'espèce *Bifidobacterium longum,* pour la préparation d'une composition thérapeutique destinée à prévenir et/ou traiter les peaux grasses ou à tendance grasse et les désordres cutanés associés.

5. Utilisation selon l'une quelconque des revendications précédentes dans laquelle ledit lysat comprend de 0,1 à 50 % en poids, en particulier de 1 à 20 % en poids de matière(s) active(s).

6. Utilisation selon l'une quelconque des revendications précédentes, dans laquelle ledit lysat est mis en oeuvre avec un microorganisme annexe, probiotique et/ou une fraction de celui-ci, distinct dudit lysat.

7. Utilisation selon l'une quelconque des revendications précédentes dans laquelle ledit lysat est mis en oeuvre par voie topique.

8. Utilisation selon la revendication précédente, **caractérisée en ce que** ledit lysat est mis en oeuvre dans une composition se présentant sous forme d'une solution aqueuse, hydroalcoolique ou huileuse, d'une dispersion du type solution ou dispersion du type lotion ou sérum, d'une émulsion de consistance liquide ou semi-liquide du type lait, obtenue par dispersion d'une phase grasse dans une phase aqueuse (H/E) ou inversement (E/H), ou d'une suspension ou émulsion de consistance molle, semi-solide ou solide du type crème, de gel aqueux ou anhydre, ou encore d'une microémulsion, de microcapsules, de microparticules, ou de dispersions vésiculaires de type ionique et/ou non ionique.

9. Composition cosmétique et/ou dermatologique, utile pour prévenir et/ou traiter les peaux grasses ou à tendance grasse, comprenant dans un milieu physiologiquement acceptable, au moins une quantité efficace d'un lysat obtenu par désintégration aux ultra sons d'un microorganisme de l'espèce *Bifidobacterium longum,* en association avec au moins un actif anti-séborrhéique.

10. Composition selon la revendication précédente, dans laquelle le lysat est tel que défini en revendication 5.

11. Procédé cosmétique pour prévenir et/ou traiter la peau grasse ou à tendance grasse et les désordres cutanés associés chez un sujet comprenant au moins l'administration audit sujet d'une quantité efficace d'un lysat obtenu par désintégration aux ultra sons d'un microorganisme de l'espèce *Bifidobacterium longum.*

12. Procédé selon la revendication précédente, dans lequel ledit lysat est tel que défini en revendication 5.

## Patentansprüche

1. Nicht-therapeutische Verwendung einer wirksamen Menge eines durch Ultraschallaufschluss eines Mikroorganismus der Gattung *Bifidobacterium longum* erhaltenen Lysats als Wirkstoff, um fettige oder tendenziös fettige Haut und damit einhergehende Hautstörungen zu behandeln und/oder zu verhindern.

2. Verwendung nach dem vorhergehenden Anspruch, **dadurch gekennzeichnet, dass** die Hautstörungen Unzulänglichkeiten der fettigen oder tendenziös fettigen Haut und/oder Hautunzulänglichkeiten vom Typ matte Haut, glänzende Haut oder irritierte Haut, Verfärbung, Rötungen, spröde Haut, gegebenenfalls mit Schuppen von trockener Haut sein können.

3. Verwendung einer wirksamen Menge eines durch Ultraschallaufschluss eines Mikroorganismus der Gattung *Bifidobacterium longum* erhaltenen Lysats als Wirkstoff zur Herstellung einer Zusammensetzung, die zur Regulierung von Seborrhoe bestimmt ist.

4. Verwendung einer wirksamen Menge eines durch Ultraschallaufschluss eines Mikroorganismus der Gattung *Bifidobacterium longum* erhaltenen Lysats zur Herstellung einer therapeutischen Zusammensetzung, die zur Vorbeugung und/oder Behandlung von fettiger oder tendenziös fettiger Haut und von damit einhergehenden Hautstörungen bestimmt ist.

5. Verwendung nach einem der vorhergehenden Ansprüche, wobei das Lysat 0,1 bis 50 Gew.-%, insbesondere 1 bis 20 Gew.-% wirksame Substanz(en) umfasst.

6. Verwendung nach einem der vorhergehenden Ansprüche, wobei das Lysat mit einem zugehörigen probiotischen Mikroorganismus und/oder einer Fraktion davon, die von dem Lysat verschieden ist, eingesetzt wird.

7. Verwendung nach einem der vorhergehenden Ansprüche, wobei das Lysat topisch eingesetzt wird.

8. Verwendung nach dem vorhergehenden Anspruch, **dadurch gekennzeichnet, dass** das Lysat in einer Zusammensetzung eingesetzt wird, die in Form einer wässrigen, hydroalkoholischen oder öligen Lösung, einer Dispersion vom Lösungstyp oder einer Dispersion vom Lotion- oder Serumtyp, einer Emulsion von flüssiger oder halbflüssiger Konsistenz vom Milchtyp, die durch Dispersion einer Fettphase in einer wässrigen Phase (H/E) oder umgekehrt (E/H) erhalten wird, oder einer Suspension oder Emulsion von weicher Konsistenz, halbfester Konsistenz oder fester Konsistenz vom Cremetyp, wässrigen Geltyp oder wasserfreien Geltyp, oder auch einer Mikroemulsion, von Mikrokapseln, Mikropartikeln oder ionischen und/oder nicht-ionischen vesikulären Dispersionen dargereicht wird.

9. Kosmetische und/oder dermatologische Zusammensetzung, die zur Verhinderung und/oder Behandlung von fettiger oder tendenziös fettiger Haut geeignet ist, umfassend, in einem physiologisch verträglichen Medium, mindestens eine wirksame Menge eines durch Ultraschallaufschluss eines Mikroorganismus der Gattung *Bifidobacterium longum* erhaltenen Lysats in Kombination mit mindestens einem Wirkstoff gegen Seborrhoe.

10. Zusammensetzung nach dem vorhergehenden Anspruch, wobei das Lysat wie in Anspruch 5 definiert ist.

11. Kosmetisches Verfahren zur Verhinderung und/oder Behandlung der fettigen oder tendenzös fettigen Haut und damit einhergehender Hautstörungen bei einer Person, umfassend mindestens die Verabreichung an die Person einer wirksamen Menge eines durch Ultraschallaufschluss eines Mikroorganismus der Gattung *Bifidobacterium longum* erhaltenen Lysats.

12. Verfahren nach dem vorhergehenden Anspruch, wobei das Lysat wie in Anspruch 5 definiert ist.

## Claims

1. The non-therapeutic use of an effective amount of a lysate obtained by disintegration with ultrasonic waves of a micro-organism of the *Bifidobacterium longum* species, as an active ingredient, for treating and/or preventing fatty skins or with a fatty tendency and the associated skin disorders.

2. The use according to the preceding claim, **characterized in that** the skin disorders may be defects of fatty skin or with a fatty tendency and/or skin defects of the dull, shiny or blotchy skin type, dyschromia, red spots, rough skins with, if appropriate, dry skin patches.

3. The use of an effective amount of a lysate obtained by disintegration with ultrasonic waves of a micro-organism of the *Bifidobacterium longum* species, as an active ingredient for preparing a composition intended to regulate seborrhea.

4. The use of an effective amount of a lysate obtained by disintegration with ultrasonic waves of a micro-organism of the *Bifidobacterium longum* species, for preparing a therapeutic composition intended to prevent and/or treat fatty skins or with a fatty tendency and the associated skin disorders.

5. The use according to any of the preceding claims, wherein said lysate comprises from 0.1 to 50% by weight, in particular from 1 to 20% by weight of active material(s).

6. The use according to any of the preceding claims, wherein said lysate is used with an ancillary, probiotic micro-organism and/or a fraction thereof, distinct from said lysate.

7. The use according to any of the preceding claims, wherein said lysate is applied topically.

8. The use according to the preceding claim, **characterized in that** said lysate is used in a composition appearing as an aqueous, hydroalcoholic or oily solution, a dispersion of the solution type or a dispersion of the lotion or serum type, an emulsion with liquid or semi-liquid consistency of the milk type, obtained by dispersing a fatty phase in an aqueous phase (O/W) or vice versa (W/O), or a suspension or emulsion of soft, semi-solid or solid consistency of the cream type of an aqueous or anhydrous gel, or further a micro-emulsion, micro-capsules, microparticles, or vesicle dispersions of the ionic and/or non-ionic type.

9. A cosmetic and/or dermatological composition, useful for preventing and/or treating fatty skins or with a fatty tendency, comprising in a physiologically acceptable medium, at least one effective amount of a lysate obtained by disintegration with ultrasonic waves of a micro-organism of the *Bifidobacterium longum* species, in association with at least one anti-seborrheic active ingredient.

10. The composition according to the preceding claim, wherein the lysate is as defined in claim 5.

11. A cosmetic method for preventing and/or treating fatty skin or with a fatty tendency, and the associated skin disorders, in a subject comprising at least the administration to said subject of an effective amount of a lysate obtained by disintegration with ultrasonic waves of a micro-organism of the *Bifidobacterium longum* species.

12. The method according to the preceding claim, wherein said lysate is as defined in claim 5.
